(19) Europäisches Patentamt / European Patent Office / Office européen des brevets

(11) **EP 2 103 664 B2**

(12) **NEW EUROPEAN PATENT SPECIFICATION**
After opposition procedure

(45) Date of publication and mention
of the opposition decision:
**05.06.2019 Bulletin 2019/23**

(45) Mention of the grant of the patent:
**22.06.2011 Bulletin 2011/25**

(21) Application number: **08005027.1**

(22) Date of filing: **18.03.2008**

(51) Int Cl.:
*C09J 7/02* (2006.01)     *A61F 13/15* (2006.01)

(54) **Release sheet material**

Trennfolienmaterial

Matériau de feuille de dégagement

(84) Designated Contracting States:
**AT BE BG CH CY CZ DE DK EE ES FI FR GB GR
HR HU IE IS IT LI LT LU LV MC MT NL NO PL PT
RO SE SI SK TR**

(43) Date of publication of application:
**23.09.2009 Bulletin 2009/39**

(73) Proprietor: **The Procter & Gamble Company
Cincinnati, OH 45202 (US)**

(72) Inventors:
• **Toro, Carlo**
**65012 Cepagatti (Pescara) (IT)**
• **Salone, Fiorello**
**65124 Pescara (IT)**
• **Pompei, Enzo**
**65129 Pescara (IT)**
• **Cataldo, Giovanni**
**66100 Chieti (IT)**
• **Carvelli, Angela**
**65123 Pescara (IT)**

(74) Representative: **Kremer, Véronique Marie
Joséphine
Procter & Gamble Service GmbH
IP Department
Sulzbacher Straße 40-50
65824 Schwalbach am Taunus (DE)**

(56) References cited:
EP-A- 1 332 746     EP-A- 1 354 575
EP-A1- 2 103 664     WO-A1-03/028609
GB-A- 2 298 627     US-A- 4 556 146

EP 2 103 664 B2

## Description

FIELD OF THE INVENTION

The present invention relates to improved release sheet materials for use in connection with disposable absorbent articles, for example sanitary napkins and the like, which are typically individually packaged prior to use.

BACKGROUND OF THE INVENTION

Disposable absorbent articles of personal hygiene are known in the art. Typical examples include sanitary napkins, panty liners, adult incontinence articles, infant diapers, paper towels, bath tissue and facial tissue. Such articles arc often used to absorb and retain bodily fluids and other exudates exereted by the human body. Many disposable absorbent articles have the same basic structure: an absorbent core encased between a liquid permeable, user contacting topsheet, which permits liquid to penetrate its thickness and contact the absorbent core where liquid is retained, and a backsheet, which may he liquid impermeable.

While there are a great many variations in the specific structural features of disposable absorbent articles, they arc typically presented to consumers in the same manner, essentially, the disposable absorbent article, irrespective of the specific structural features used, is packaged in a box or hag from which the consumer withdraws the article, as needed. In order to protect the article from soiling or contamination from the time it is removed from the box or bag until the article is used, for example if a woman wanted to carry a sanitary napkin, with her for use away from home, the articles may be individually packaged within the box or bag by means of a sheet of material which is wrapped around the individual article.

A typical individual package for disposable absorbent articles is discloses, for example, in US patent 4,556,146, which describes a disposable absorbent article, typically a sanitary napkin, associated with a wrapper which overlays one major surface of the sanitary napkin. The wrapper extends beyond the perimeter of the disposable absorbent article so that when the disputable absorbent article and the wrapper are folded as a unit, the longitudinal side flaps of the wrapper, which extend beyond the longitudinal sides of the article, may be frangibly sealed thereby providing the disposable absorbent article with an individual package It is also common to provide the disposable absorbent article with an adhesive element on the garment facing side of the backsheet which, in use, serves to affix the absorbent article to the wearer's undergarment thereby maintaining the absorbent article in place against the wearer's body. The adhesive clement may take the form of a coating of adhesive which is in strips or other suitable pattern. For example, the garment facing side of the backsheet can be coated uniformly with a layer of pressure sensitize hot melt adhesive. The wrapper overlays the garment facing side of the backsheet with the longitudinal flap portions extending beyond the longitudinal perimeter segments of the absorbent article. The wrapper typically is not folded onto or otherwise brought into contact with the body facing side of the topsheet; in other words, the surface of the wrapper facing the garment facing side of the backsheet is in face to face relation substantially with said side only of the backsheet. The wrapper is typically releasably affixed to the disposable absorbent article, e.g. a sanitary napkin, by the aforementioned adhesive element. When an adhesive element is used in this manner, it is not necessary to provide the absorbent articles with a separate release sheet in order to protect the adhesive element before use, as this function is provided by the wrapper.

To individually packaged the absorbent article, the article and the affixed wrapper can be typically folded as a unit. That is, they are folded together with the wrapper remaining in place with respect to the absorbent article. Typically, the absorbent article is folded lengthwise into thirds about two fold axes. The longitudinal side flaps or flap portions of the wrapper are frangibly sealed using any of the well known sealing techniques. For example, the longitudinal flap portions may be heat sealed, glued, ultrasonically bonded, or crimped.

In use, the individually packaged absorbent article is provided to a user. The user may then break the frangible seals, unfold the wrapper/absorbent article unit and separate the wrapper from the absorbent article, for example a sanitary napkin, exposing the adhesive clement. The absorbent article may then be used as such devices normally are, typically being adhered by means of the adhesive element to the crotch portion of an undergarment, which is subsequently worn.

An advantage of an individually packaged disposable absorbent article, for example a sanitary napkin or a pantiliner, is discreetness, as a user does not need to take with her an entire box or bag of products, but only a small number of individually packaged articles, as needed for subsequent use, for example when staying away from home. However, materials commonly used for the wrapper, typically flexible materials which can be also liquid impermeable such as polymeric films, for example polyethylene films which can be selected for low cost and case of processability, can be rather noisy when the packaged product is manipulated in order to unfold the wrapper with the absorbent article, breaking the seals along the longitudinal flap portions of the wrapper, and then separating the article from the wrapper itself by detaching the adhesive element. This is typically due to the nature of the wrapper material, namely its composition, and to its relatively high thickness, usually around 20-30 $\mu$, for example 25 $\mu$, which is dictated by process reasons. In a high speed production line the material of the wrapper is typically provided in a continuous web, the adhesive element may he provided in selected areas directly onto the wrapper material continuous web, and then absorbent articles are provided onto each respective adhesive clement, by adhering thereon the garment facing side of the backsheet. The wrapper material continuous web may be folded together with the applied absorbent article, and cut and sealed in order to form individually packaged articles. When the user, after unfolding the packaged and breaking the seals, separates the absorbent article from the wrapper material, at least some of the adhesive element remain on the garment facing surface of the backshcct since the respective surface of the wrapper element is typically provided with means having an inferior adhesion to the adhesive material.

Typically the wrapper material can include a coating of a silicone composition. Silicone coating can he typically provided by applying to the sheet material used for the wrapper a layer of a silicone precursor composition, for example comprising a crosslinkable organosilicon prepolymer, which is then cured, namely crosslinked, and anchored to the sheet material surface. Curing/crosslinking can be typically performed by providing energy to the precursor composition in presence of a suitable catalyst or initiator, for example the so called UV curing process is typically performed by providing UV radiation in the presence of a photoinitiator, and thermal curing involves provision of heat in the presence of a catalyst. While UV curing is a relatively straightforward process, it is based on a more complex chemistry, and typically requires rather expensive silicone precursor compositions for successful curing.

Thermal curing on the other hand can be potentially more convenient, as precursor materials are typically simpler and less expensive, and moreover a lesser amount of said precursor composition is typically necessary in order to apply to a certain sheet material, e.g. a polymer film, a silicone coating providing a same release capability towards adhesives commonly used as panty fastening adhesives in sanitary absorbent articles.

However, the heat typically necessary to effectively crosslink the precursor composition, in order to finally provide the silicone coating, can be typically incompatible with low cost, low thickness polymeric films which can be selected as sheet material for the wrapper, for example polyethylene films, or polyethylene based films, which can he selected for their low cost and ease of processability. A polyethylene, or polyethylene based film material having n relatively low thickness, which is desirable in terms of discreetness of the final product, as explained above, as well as for cost reasons, cannot typically withstand the thermal stress provided by the thermal curing step of the silicone precursor composition, and can shrink or deform, or also melt.

It is therefore desirable to provided a flexible release sheet material tor use as wrapper material for disposable absorbent articles, which is more discreet, particularly less noisy upon use, than known products, and at the same time comprises an effective layer of a silicone coating achieved by means of simple thermal curing methods.

## SUMMARY OF THE INVENTION

The present invention addresses the above need by providing a flexible release sheet material being a polyolefin film comprising a layer of thermal cured silicone based release composition.

The sheet material has a basis weight of from 1 $g/m^2$ to 25 $g/m^2$, preferably of less than 20 $g/m^2$ more preferably of less than 18 $g/m^2$, even more preferably of from 5 $g/m^2$ to 15 $g/m^2$, and comprises at least 50% by weight of polyethylene having at least 10% crystallinity.

## BRIEF DESCRIPTION OF THE DRAWINGS

Figure 1 is a partially cut away perspective view of an absorbent article and its associated wrapper prior to being folded and sealed.

Figure 2 is a partially cut away perspective view of an absorbent article and its associated wrapper after they have been folded and sealed to form an individually packaged absorbent article.

## DETAILED DESCRIPTION OF THE INVENTION

Referring now to the drawings there is shown an individually packaged disposable absorbent article embodying the teachings of the present invention. As used herein the term "absorbent article" refers to those articles intended to absorb and retain liquid and in particular to those articles which are placed against or in proximity to a wearer's body to absorb and contain the various liquids discharge from the body (e.g. blood, menses, urine). A "disposable, absorbent article" is an absorbent article which is intended to be discarded after a single use (i.e., they are not intended to be laundered or otherwise restored and reused). Typical disposable absorbent articles according to the present invention can be absorbent articles for feminine hygiene such as sanitary napkins and panty liners, commonly referred to collectively as catamenial pads, light incontinence products, or the like.

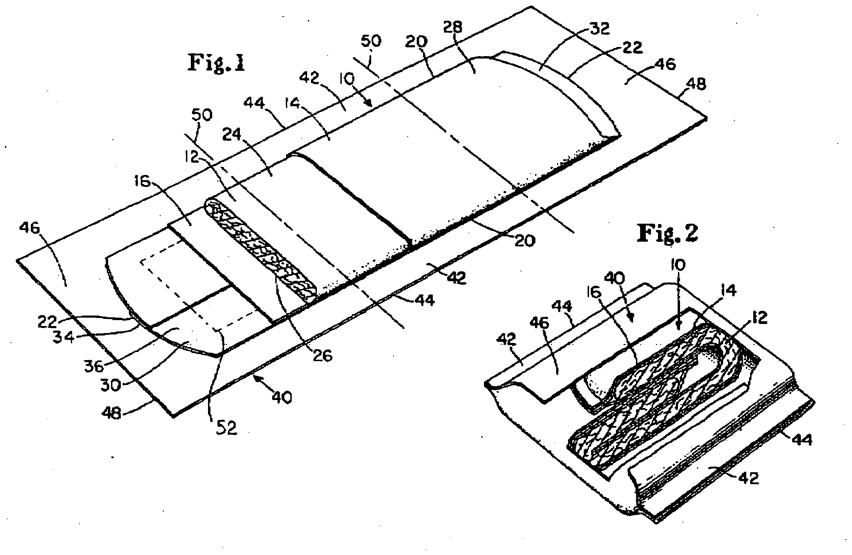

FIG. 1 is a partially cut away perspective view of a catamenial pad 10 and a wrapper 40 prior to being folded and sealed as set forth in greater detail herein below. A catamenial pad is a disposable absorbent article which is worn by females external to and in the proximity of the urogenital region and which is intended to absorb and contain menstrual fluids and other vaginal discharges. As used herein the term "catamenial pad" includes pantiliners which are often worn by females external to the urogenital region between periods of heavy menstrual flow and which are intended to absorb light menstrual flow and nonmenstrual vaginal discharges. The primary difference between catamenial pads used during periods of heavy menstrual flow and catamenial pads used between periods of heavy menstrual flow (i.e., pantiliners) is the absorbent capacity of the pad.

While the present invention will be described with reference to a catamenial pad, it should be understand that the present invention has application in the context of other disposable absorbent articles such as, for example, light incontinence products. Further, the teachings of this invention have application to catamenial pads manufactured according to the teachings of any of the multitudinous references in the catamenial pad art. A typical catamenial pad embodiment will now be described briefly.

As can be seen in FIG. 1, the catamenial pad 10 basically can comprise an absorbent pad 12, an envelope sheet 14 and a barrier 16. The catamenial pad 10 has a perimeter generally comprising longitudinal perimeter segments, or longitudinal sides 20 and transverse perimeter segments, or transverse ends 22. The perimeter defines the outer boundary of the catamenial pad 10, while the longitudinal perimeter segments 20 and the transverse perimeter segments 22 define the outer boundary of the catamenial pad 10 along each longitudinal side and each transverse end respectively.

The absorbent pad 12 is generally compressible, conformable, and non-irritating to the user's skin and may be manufactured from a wide variety of absorbent materials which are capable of absorbing and retaining liquids. For example, a hatt of absorbent fibers, a multiplicity of plies of creped cellulose wadding, or any equivalent material may be used. The absorbent capacity of the material used, however should be sufficient to absorb and retain the expected liquid loading in the intended use of the absorbent article without undue bulk. An example of a suitable catamenial pad 10 intended to receive heavy menstrual discharges of approximately 40 millilitres, may include about 8 grams of comminuted wood pulp, generally referred to as airfelt.

The shape and dimensions of the absorbent pad 12 can be selected to permit the disposable absorbent article to conform to and lit about the portion of the body against which it will be placed. Often, as in the embodiment illustrated, the general shape and dimensions of the catamenial pad 10 can he determined by the shape and dimensions of the absorbent pad 12. In the embodiment illustrated in the figures, the shape and dimensions of the absorbent pad 12 were selected to permit the catamenial pad 10 to conform to the urogenital region of the wearer's body. While the shape and dimensions of the absorbent pad 12 may be varied, it has been found that a generally planar configuration having a first major surface, or body facing side, 24 and a second major surface, or garment facing side, 26 can be suitable. The first major surface 24 is that surface of the absorbent pad 12 facing toward the source of liquid (i.e. toward the wearer's body) and the second major surface 26 is that surface of the absorbent pad 12 facing away from the source of liquid. An example of a suitable catamenial pad 10 can have a generally rectangular, planar shaped absorbent pad 12 having a length of about 9.0 inches (22.9 centimetres) and a width of about 2.5 inches (6.4 centimetres). It should be understood, however, that other shapes (e.g. elongated ovals, triangles, squares, etc.) and other dimensions may be used.

The catamenial pad 10 may include an envelope sheet 14 that encases the absorbent pad 12 and is preferably compliant, soft feeling, and non-irritating to the wearer's body. The envelope sheet 14 can help maintain the structural integrity of the absorbent pad 12 and has a first and a second end flap 30 and 32 respectively. The envelope sheet 14 may be wrapped about the absorbent pad 12 and may be affixed to itself along a seam 34 which is adjacent the second major surface 26 and which traverses the catamenial pad 10 longitudinally. The first and second flaps 30 and 32 respectively extend beyond the transverse ends of the absorbent pad 12 and are typically sealed so as to completely encase the absorbent pad 12 within the envelope sheet 14. The portion of the envelope sheet 14 overlaying the first major surface 24 is the topsheet portion 28 corresponding to the body facing side of the article, and the portion of the envelope sheet 14 overlaying the second major surface 26 is the backsheet portion 36 of the envelope sheet 14, corresponding to the garment facing side of the article. The topsheet portion 28 is liquid permeable. In use the topsheet portion 28 may contact the skin of the catamenial pad wearer and permit the transmission of liquid through its thickness to the absorbent pad 12 where the liquid may be retained.

There are many suitable materials from which the envelope sheet 14 may he manufactured. The topsheet portion 28 may be manufactured from either hydrophobic or hydrophilic fibres and may, for example, be cared, spun bonded, melt blown, or air laid. Alternatively, the topsheet portion 28 may be a continuous film or sheet of, for example, thermoplastic material which is apertured. A suitable topsheet portion 28 is described in U.S. Pat. No. 4,324,246 which issued to Mullane et al on Apr. 13, 1982.

The topsheet portion 28 and the backsheet portion 36 may either be integral (i.e. the backsheet portion 36 and the topsheet portion 28 are separate elements affixed to each other) or unitary (i.e. the backsheet portion 36 and the topsheet portion 28 are formed from a continuous and undivided sheet of material) and may either have the same nr different physical properties. The embodiment of FIG. 1 shows the topsheet portion 28 and the backsheet portion 36 as being unitary.

To help prevent liquids absorbed by the absorbent pad 12 from penetrating through the backshcct portion 36, it may he advantageous to interpose a barrier 16 at the interface between the second major surface 26 of the absorbent pad 12 and the backsheet portion 36. The barrier 16 may be manufactured from any flexible, liquid impermeable material which is non-irritating to the wearer. In certain embodiments, the barrier 16 may be a sheet of polyethylene which is coincident with the backsheet portion 36.

Alternatively, the envelope sheet 14 may comprise a topsheet portion 28 and a backsheet portion 36 which are made integral with each other by affixing them together about their periphery. In such embodiments, the topsheet portion 28 may be at least partially liquid impervious and the backsheet portion 36 may be liquid pervious or wholly or partially liquid impervious.

It is common to provide the catamenial pad 10 with an adhesive element 52, partially shown in dotted line in FIG. 1. The adhesive element 52 is positioned on the garment facing side of the article, namely on the backsheet portion 36 and, in use, serves to affix the catamenial pad 10 to the wearer's undergarments thereby maintaining the catamenial pad 10 in place against the wearer's body. The adhesive element 52 may take the form of a coating of adhesive which is in strips or any other suitable pattern The backsheet portion 36 may be coated uniformly with a layer of a pressure sensitive hot melt adhesive such as, for example. NS34-2R23 as manufactured by National Starch and Chemical of Bridgewater, N.J.

In accordance with the teachings of this invention, a wrapper 40 is associated with, and typically can have dimensions generally larger than those of the catamenial pad 10 Thus, the wrapper 40 has longitudinal flap portions 42 comprising that portion of the wrapper 40 between the longitudinal edge 44 of the wrapper and the longitudinal perimeter segment or longitudinal side 20 of the catamenial pad 10. In the embodiment illustrated in FIG. 1, the wrapper 40 also has transverse flap portions 46 comprising that portion of the wrapper 40 between the transverse edges 48 of the wrapper 40 and the transverse perimeter segments or transverse ends 22 of the catamenial pad 10.

The wrapper 40 is shown overlaying the garment facing side of the absorbent article, namely the backsheet portion 36, with the longitudinal flap portions 42 typically extending beyond the longitudinal perimeter segments 20. In the absorbent shown in FIG. 1, the wrapper 40 is not folded onto or otherwise brought into contact with the topsheet portion 28. In other words, the surface of the wrapper 40 facing the backsheet portion 36 is in face to face relation with the backsheet portion 36 only. The wrapper 40 is releasably affixed to the catamenial pad 10 by the aforementioned adhesive element 52; hence it is also defined as a releasable wrapper 40 according to the present invention. If an adhesive element is use in this manner, it is not necessary to provide the absorbent article with a separate release sheet as is commonly done in prior art devices, as the function of protecting the adhesive element from contamination prior to use is provided by the wrapper.

To individually packaged the catamenial pad 10, the catamenial pad 10 and the affixed wrapper 40 can be folded as a unit. That is, they can be folded together with the wrapper 40 remaining in place with respect to the catamenial pad 10. According to the present invention, the absorbent articles and the wrapper can be folded as a unit about at least one fold-axis, e.g. a longitudinal or a transverse fold-axis, in order to define a packaged absorbent article. In certain embodiments, the catamenial pad 10 may be folded lengthwise into thirds about two tranverse fold-axes 50, as shown in FIG. 2, to define a packaged comprising the absorbent article. The longitudinal flap portions 42 can be frangibly sealed using any of the well-known scaling techniques. For example the longitudinal flap portions 42 may be heat sealed, glued, or ultrasonically bonded.

In use, the individually packaged catamenial pad is provided to a user. The user may then break the seals, unfold the catamenial pad 10, and separate the wrapper 40 from the catamenial pad 10. The catamenial pad 10 may then be used as such devices normally are.

Means having a reduced adhesion to the adhesive material of the adhesive element 52 can be typically provided to the material or the wrapper 40, namely on the surface which is meant to contact and he affixed to, the adhesive element 52 on the garment facing surface of the absorbent article.

Means for providing the material of the wrapper 40 with a reduced adhesion to the adhesive material can typically comprise silicone release coatings. Silicones typically otter excellent release propertied; in order to present a non-stick surface and, at the same time, offer little or no transfer to the released surface, should form a cured film-like surface free from migratory species Suitable silicone polymers can be selected, such as polysiloxanes, for example polydimethylsiloxanes (PDMS), and are typically coated according to usual techniques onto the surface of the material to be treated, for example as a solution, or an emulsion, or 100 percent solid, in the desired amount or basis weight. In order to tie all the polymer chains together and form a coherent film, well anchored on the material substrate, crosslinking, or curing, of the silicone polymer is performed. According to the present invention thermal curing can be typically used as it allows the achievement of a same release effect towards the typical adhesive materials used in absorbent articles, such as for example the adhesive element 52 in the catamenial pad 10 illustrated in Figure 1. with a reduced amount of silicone polymer, compared to other crosslinking or curing methods, for example by means of UV radiation. Application of heat may he provided by microwaves, or infrared, or in a hot forced air oven, as typical. Amount of necessary heat can depend on the amount of silicone polymer adopted, and on the desired degree of crosslinking in order to have a coherent release film. In a typical process the substrate with the silicone coating, usually as a continuous sheet material, runs through the oven with a selected speed, in order to achieve the desired heating to a selected temperature, and consequently the curing/crosslinking, of the silicone polymer. Typically the thermal curing is performed in the presence of a suitable metal catalyst, for example a platinum-based catalyst, provided in the necessary amount. It is necessary that the substrate material where the silicone release coating has to he provided, typically the releasable wrapper 40, withstands the heating and hence the temperatures involved in the thermal curing without deforming or melting, as this would negatively effect the entire process and the final product.

According to the present invention, the releasable wrapper 40 can be typically provided by a flexible release sheet material being a polyolefin film comprising a layer of thermal cured silicone based release composition, wherein the flexible release sheet material is particularly thin having a basis weight of from 1 $g/m^2$ to 25 $g/m^2$, or of less than 20 $g/m^2$, or of less than 18 $g/m^2$, or also from 5 $g/m^2$ to 15$g/m^2$. The presence of a major percentage of polyethylene in the polyolefin film, namely at least 50% by weight, is due to the good mechanical properties of polyethylene, combined with compliance and flexibility which provide discreetness and soft feeling during use, further, polyethylene has a relatively low cost. It has been discovered that the selected degree of crystallinity in the polyethylene surprisingly provides the film, despite its reduced thickness, with sufficient heat resistance to withstand thermal curing of the silicone based release composition applied thereto. If the polyethylene content increases in the composition of the polyolefin film providing the flexible release sheet material of the present invention, the respective degree of crystallinity shall typically also increase. For a film substantially made of polyethylene, the respective degree of crystallinity can he at least 30%. Degree of crystallinity, or simply crystallinity, of polyethylene, or of the polyethylene traction in a polyolefin film, is a common paremeter, readily available from film suppliers, or measurable with techniques known in the art. It indicates the weight percent of crystalline polyethylene in the total polyethylene content of the sheet material, typically a film.

According to the present invention, only a rather low amount of thermal cured silicone polymer can be provided to the flexible release sheet material in order to have an effective release action towards for example the adhesive compositions which can be typically used as panty fastening adhesives in absorbent articles, when the flexible release sheet materials is used as a releasable wrapper for said absorbent articles. Hence according to an embodiment of the present invention the layer of thermal cured silicone based release composition can be typically provided in a basis weight of less than 1.0 $g/m^2$, or from 0.2 $g/m^2$ to 0.8 $g/m^2$, or also from 0.4 $g/m^2$ to 0.6 $g/m^2$.

The flexible release sheet material, having the selected combination of low basis weight, which transtates into a low thickness, and degree of cryslallinity of the polyethylene component, can provide the desired characteristics of softness and flexibility, and the necessary heat resistance to withstand the thermal curing step upon provision of a layer of a silicone release composition. Heat resistance and low thickness in addition allow the flexible release sheet material to be effectively processed in a high speed production line in order to be provided with a thermal cured silicone based release composition, with no disruptions in the process, and without compromising the quality of the final product.

According to an embodiment of the present invention, the flexible release sheet material can be selected among polymeric films having a thickness of 2-26 $\mu$, or of less than 21 $\mu$, or also of less than 19 $\mu$, or even of 6-16 $\mu$.

Single layer or multilayer polymeric films can he selected for the wrapper material according to the present invention, which are typically obtainable for example through a single layer extrusion or a multilayer co-extrusion process. As known, the only layer, or each layer of the polymeric film can comprise a single polymer or a blend of different polymers.

According to an embodiment of the present invention, the polymeric film for the flexible release sheet material can comprise at least 0.5% by weight, or at least 3% by weight, and up to a maximum of 50% by weight, or of 20% by weight, or of 10% by weight, of a further polymer or copolymer having a melting point which is higher than that of the polyethylene component. Typically this further polymer can he polypropylene. It has been discovered that films comprising such polyethylene/polypropylene blends can provide even improved heat resistance with a particularly low basis weight and thickness; polypropylene can also improve mechanical resistance of said polyethylene-based films.

According to a further embodiment of the prevent invention, the polymeric film for the flexible release sheet material can also comprise an inert component typically in particulate or powder form. Suitable inert components can be comprised in an amount of 0.5% to 40% in weight, or of 3% to 30% by weight, or of 10% to 20% by weight of the finished polymeric film. The inert component, typically in particulate or powder form, can comprise calcium carbonate ($CaCO_3$), or also kaolin, diatomaccous earth, or mixtures thereof. Calcium carbonate can be typically available from chalk or limestone. The presence of an mert component, typically calcium carbonate, can increase the physical properties of the polymeric film, particularly heat resistance, which can be beneficial in the context of the present invention, where a thin polymeric film as flexible release sheet material is provided with a silicone based release composition by thermal curing. Also inclusion of an inert component in the formulation of a polymeric film for the flexile release material of the invention can lower the final cost of the material itself.

As explained above, thermal curing of a silicone based release composition can be usually performed in the presence of a suitable metal catalyst, typically a platinum catalyst. Metal, typically platinum catalysts are sensitive to contamination by certain compounds which may stop or inhibit cure, and should therefore be presented from contacting the catalyst during the curing step. Catalyst potential poisons or inhibitors can comprise compounds containing nitrogen, namely amines and amides such as neutralizing amines, ethanolamine, N-methylethanolamine, triethanolamine, N,N-dimethyl ethanolamine, n-butylamine, diethylamine, triethylamine, tetramethylenediamine, cyclohexylamine, melamine, dimethylformamide, or nitrides, cyanates, oximo, nitroso, hydrazo, azo compounds such as adiponitrile, 2-butoxime, alpha-nitroso-beta-napthol, or chelates such as EDTA (ethylenediaminetetracetic acid), NTA (nitiloacetic acid), compounds containing sulfur, namely sulfides, thio compounds such as dibenzyldisulfide, thioacetic acid, allylthiourea; compounds containing tin, namely fatty acid tin salts such as those used in tin-catalyzed silicone release coatings; compounds containing phosphorous, namely phosphines such as triphenylphosphine, phosphines such as triethylphosphite. The amount of possible catalyst poisons or inhibitors, for example in the sheet material itself, should be typically kept at the lowest possible level, in order of not impair the curing process.

In addition to a suitable crosslinking/curing of the silicone based release composition provided onto the flexible release sheet material of the present invention, in order to form a coherent film, an effective anchoring onto the sheet material itself can he also useful in order to provide a release surface which does not allow portions of the silicone based composition to migrate, for example into the adhesive composition meant to contact, in the packaged product, the flexible release sheet; this effect could in fact alter the characteristics of the adhesive, and for example reduce its adhesion, which may he detrimental for the subsequent intended use of the absorbent article.

Good anchoring of the thermal cured silicone based release composition to the surface of the fexible release sheet material can be influenced by surface characteristics of the sheet material per se. It could for example be possible to improve the surface characteristics of the flexible release sheet material by means of a corona treatment, before application of the silicone based release composition to be subsequently thermal cured.

A measure of the effective crosslinking/curing, as well as anchoring, of the thermal cured silicone based release composition applied onto the flexible release sheet material of the present invention can be the extractable fraction of said silicone based release composition, which corresponds to the amount of silicone which can be extracted from the release coating according to a suitable method. According to an embodiment of the present invention, a flexible release sheet material can have a thermal cured silicone based release composition with an extractable fraction of less than 15%, preferably of less than 10%, more preferably of less than 5%, by weight of the thermal cured silicone based release composition, wherein the extractable fraction is evaluated according to the method "Extractables in silicone release coatings" as described herein.

According to a further embodiment of the present invention; the flexible release sheet material may be selected in order to have a relatively high coefficient of motion, typically on the surface opposite to that subjected to the low adhesion treatment, hence typically on the surface without the silicone based release coating. A sufficiently high coefficient of friction of the wrapper material may be desirably in high speed manufacturing processes, as it many help prevent that the very thin and light material, both when still in the form of a continuous material web, and particularly after it is already in the form of single bags, from slipping during processing, and can add to its stability. The coefficient of friction of the wrapper material can be evaluated with the standard test method ASTM D 1894-06, as described in details in the "Test Methods" section below. According to this embodiment of the present invention, the coefficient of friction of the wrapper material, typically onto the surface opposite to that interested by the low adhesion treatment (i.e. the silicone coating) is comprised between 0.1 and 0.7, or between 0.2 and 0.6, or even also between 0.3 and 0.5.

An individually packaged absorbent article using as releasable wrapper the flexible release sheet material according to the present invention can guarantee a greatly increased discreetness to the user, as the selected wrapper material is much less noisy than current materials when manipulated, typically when the user breaks the seals to open the package, and then separates the absorbent article from the wrapper itself, in order to prepare the absorbent article for its intended use by exposing the adhesive element for attachment to the undergarment. Also the simple handling of the packaged product is quieter, providing the user with better self-confidence and tranquility. At the same time, the individually packaged absorbent article of the present invention can be manufactured with known high speed processes, with no need of drastic adjustments or changes.

Finally the individually packaged absorbent article of the present invention is also cheaper, as it uses a sensibly lighter material for the releasable wrapper, and moreover can be provided with a release coating of a silicone based release composition which is thermal cured, and hence necessitating of a lower amount of release composition compared to what needed with other traditional curing methods, for example by means of UV radiation.

Test Methods.

Extractables in silicone release coatings.

The percent of extractables is representative of the amount of uncrosslinked silicone that disengages from a cured release-coated sample in the presence of a solvent; it is evaluated according to the present method as percentage of extractable silicon, referred to herein as extractable traction. The lower the extractable fraction, the more complete and effective the cure. The extractable action of a cured silicone release coaling may he determined using the following procedure designed for an Oxford Lab-X 3000XRF (X-Ray Fluorescence) analyzer, but can be suitably adapted by the skilled person for other manufacturers' benchtop XRF instruments,

1. Cut three sample discs from the substrate using a sample punch and place the discs on a clean sheet of paper; use tweezers to handle the discs at all times. The disc diameter can be selected depending on the size of the substrate, for example a diameter of 30 mm should be suitable in most cases. Samples should be adequately conditioned for at least 24 hours at 25°C and 50% relative humidity.

2. Determine the silicone coat weight on each sample using the Oxford Instruments Lab-X 3000 XRF analyzer; a sample spinner is typically required on the Lab-X 3000 to give the most accurate results.

3. Place the three discs in a 100 ml bottle containing 40 ml of methylisobutyl ketone solvent; seal the bottle and put it on a mixing wheel for 30 minutes; after 30 minutes, remove the discs from the bottle using tweezers and place them on clean tissue paper, silicone coated surface up.

4. Allow the solvent to evaporate from the sample discs; do not wipe or blot the sample discs.

5. Allow the sample discs to air dry for ten minute or longer to ensure complete evaporation of the methylisobutyl ketone.

6. Measure the final coating weight of each sample disc as described at point 2 above.

The extractable silicone present in the in the coaling after preparation under specified conditions is determined. The quantity of extractables is expressed as a percentage of the initial coat weight.

The percent of extractables (extractable fraction) is calculated as follows:

$$\text{extractable fraction} \% = \frac{(a-b)}{a} \times 100$$

Where a = initial coat weight (before contact with methylisobutyl ketone b-final coat weight (after contact with methylisobutyl ketone)

Coefficient of friction.

The coefficient of friction of the wrapper material of the individually packaged absorbent article of the present invention is evaluated as the kinetic coefficient of friction according to the standard test method ASTM D 1894-06. The Assembly of Apparatus of Fig. 1(c) has been adopted, selecting a speed setting for a crosshead motion of 127 mm/min, but any other suitable assembly and speed setting as prescribed in Section 7 of the standard method can he chosen

Each dimension for which a value is defined herein is a technical dimension, which, in the context of the present invention is not to be understood literal. Hence, all embodiment having dimensions functionally equivalent to the dimensions stated herein are intended to be covered by the scope of the invention, e.g. a dimension of "40 mm" has to be understood as meaning "about 40 mm".

**Claims**

1. A flexible release sheet material comprising a layer of thermal cured silicone based release composition, said sheet material being a polyolefin film having a basis weight of from 1 g/m$^2$ to 25 g/m$^2$, preferably of less than 20 g/m$^2$, more preferably of less than 18 g/m$^2$, even more preferably of from 5 g/m$^2$ to 15 g/m$^2$, said sheet material comprising at least 50% by weight of polyethylene having at least 10% crystallinity.

2. A flexible release sheet material according to any preceding claim, wherein said polyolefin film has a thickness of 2-26 $\mu$, preferably of less than 21 $\mu$, more preferably of less than 19 $\mu$, even more preferably of 6-16 $\mu$.

3. A flexible release sheet material according to any preceding claim, wherein said sheet material comprises at least 0.5% by weight, preferably at least 3% by weight, of a further polymer or copolymer having a melting point higher than that of said polyethylene.

4. A flexible release sheet material according to claim 3, wherein said further polymer is polypropylene.

5. A flexible release sheet material according to claim 1, said sheet material being substantially made of polyethylene having at least 30% crystallinity.

6. A flexible release sheet material according to any preceding claim, said layer of thermal cured silicone based release composition having a basis weight of less than 1.0 g/m$^2$, preferably from 0.2 g/m$^2$ to 0.8 g/m$^2$, more preferably from 0.4 g/m$^2$ to 0.6 g/m$^2$.

7. A flexible release sheet material according to any preceding claim, said layer of thermal cured silicone based release composition having an extractable fraction of less than 15%, preferably of less than 10%, more preferably of less than 5%, by weight of said thermal cured silicone based release composition, said extractable fraction evaluated according to the method "Extractables in silicone release coatings" as described herein.

8. A releasable wrapper for an individually packaged sanitary article, said releasable wrapper comprising a flexible release sheet material according to any preceding claim.

**9.** An individually packaged sanitary article comprising an absorbent article having a body facing side, a garment facing side, two longitudinal sides and two transverse ends, said absorbent article having an adhesive element on said garment facing side, a releasable wrapper overlaying said garment facing side of said article and releasably affixed to said adhesive element, said absorbent article and said wrapper being folded as a unit about at least one fold-axis to define a package composing said absorbent article, wherein said releasable wrapper is according to claim 8.

**10.** A process for making a flexible release sheet material according to any of claims 1 to 7, comprising the steps of:
providing a sheet material comprising at least 50% by weight of polyethylene having at least 10% crystallinity, providing said sheet material with a coating of a suitable thermocurable silicone polymer, curing said silicone polymer by providing heat.

**Patentansprüche**

**1.** Flexibles Trennlagenmaterial, umfassend eine Schicht aus einer thermisch gehärteten Trennmittelzusammensetzung auf Silikonbasis, wobei das Lagenmaterial eine Polyolefinfolie mit einem Flächengewicht von 1 g/m$^2$ bis 25 g/m$^2$, vorzugsweise von weniger als 20 g/m$^2$, mehr bevorzugt von weniger als 18 g/m$^2$, noch mehr bevorzugt von 5 g/m$^2$ bis 15 g/m$^2$ ist, wobei das Lagenmaterial mindestens 50 Gew.-% Polyethylen mit einer Kristallinität von mindestens 10 % umfasst.

**2.** Flexibles Trennlagenmaterial nach einem der vorstehenden Ansprüche, wobei die Polyolefinfolie eine Dicke von 2-26 μ, vorzugsweise von weniger als 21 μ, mehr bevorzugt von weniger als 19 μ, noch mehr bevorzugt von 6-16 μ aufweist.

**3.** Flexibles Trennlagenmaterial nach einem der vorstehenden Ansprüche, wobei das Lagenmaterial zu mindestens 0,5 Gew.-%, vorzugsweise zu mindestens 3 Gew.-%, ein weiteres Polymer oder Copolymer mit einem Schmelzpunkt höher als derjenige des Polyethylens umfasst.

**4.** Flexibles Trennschichtmaterial nach Anspruch 3, wobei das weitere Polymer Polypropylen ist.

**5.** Flexibles Trennlagenmaterial nach Anspruch 1, wobei das Lagenmaterial im Wesentlichen aus Polyethylen mit einer Kristallinität von mindestens 30 % besteht.

**6.** Flexibles Trennlagenmaterial nach einem der vorstehenden Ansprüche, wobei die Schicht aus einer thermisch gehärteten Trennmittelzusammensetzung auf Silikonbasis ein Flächengewicht von weniger als 1,0 g/m$^2$, vorzugsweise von 0,2 g/m$^2$ bis 0,8 g/m$^2$, mehr bevorzugt von 0,4 g/m$^2$ bis 0,6 g/m$^2$ aufweist.

**7.** Flexibles Trennlagenmaterial nach einem der vorstehenden Ansprüche, wobei die Schicht aus einer thermisch gehärteten Trennmittelzusammensetzung auf Silikonbasis einen extrahierbaren Anteil von weniger als 15 Gew.-%, vorzugsweise von weniger als 10 Gew.-%, mehr bevorzugt von weniger als 5 Gew.-% der thermisch gehärteten Trennmittelzusammensetzung auf Silikonbasis aufweist, wobei die extrahierbare Fraktion nach dem Verfahren "Extrahierbare Substanzen in Silikontrennbeschichtungen", wie hierin beschrieben, bewertet wird.

**8.** Abziehbare Einwickelfolie für einen einzeln abgepackten Hygieneartikel, wobei die abziehbare Einwickelfolie ein flexibles Trennlagenmaterial nach einem der vorstehenden Ansprüche umfasst.

**9.** Einzeln verpackter Hygieneartikel, umfassend einen Absorptionsartikel mit einer körperzugewandten Seite, einer bekleidungszugewandten Seite, zwei Längsseiten und zwei Querenden, wobei der Absorptionsartikel ein Klebeelement auf der bekleidungszugewandten Seite aufweist, eine lösbare Schutzhülle, die die bekleidungszugewandte Seite des Artikels überdeckt und lösbar an dem Klebeelement befestigt ist, wobei der Absorptionsartikel und die Schutzhülle als Einheit um mindestens eine Faltachse gefaltet sind, um eine Verpackung zu definieren, die den Absorptionsartikel bildet, wobei die lösbare Schutzhülle nach Anspruch 8 ist.

**10.** Verfahren zum Herstellen eines flexiblen Trennlagenmaterials nach einem der Ansprüche 1 bis 7, das folgende Schritte umfasst:
Bereitstellen eines Lagenmaterials, das zu mindestens 50 Gew.-% Polyethylen mit mindestens 10 % Kristallinität umfasst, Bereitstellen des Lagenmaterials mit einer Beschichtung aus einem geeigneten wärmehärtbaren Silikonpolymer, Härten des Silikonpolymers durch Bereitstellen von Wärme.

**Revendications**

**1.** Matériau en feuille antiadhésif souple comprenant une couche de composition antiadhésive à base de silicone durcie thermiquement, ledit matériau en feuille étant un film polyoléfinique ayant une masse surfacique allant de 1 g/m$^2$ à 25 g/m$^2$, de préférence inférieure à 20 g/m$^2$, plus préférablement inférieure à 18 g/m$^2$, encore plus préférablement allant de 5

g/m$^2$ à 15 g/m$^2$, ledit matériau en feuille comprenant au moins 50 % en poids de polyéthylène ayant au moins 10 % de cristallinité.

2. Matériau en feuille antiadhésif souple selon une quelconque revendication précédente, dans lequel ledit film polyoléfinique a une épaisseur de 2 à 26 $\mu$m, de préférence inférieure à 21 $\mu$m, plus préférablement inférieure à 19 $\mu$m, encore plus préférablement de 6 à 16 $\mu$m.

3. Matériau en feuille antiadhésif souple selon l'une quelconque des revendications précédentes, dans lequel ledit matériau en feuille comprend au moins 0,5 % en poids, de préférence au moins 3 % en poids, d'un polymère ou copolymère supplémentaire ayant un point de fusion plus élevé que celui dudit polyéthylène.

4. Matériau en feuille antiadhésif souple selon la revendication 3, dans lequel ledit polymère supplémentaire est du polypropylène.

5. Matériau en feuille antiadhésif souple selon la revendication 1, ledit matériau en feuille étant essentiellement constitué de polyéthylène ayant au moins 30 % de cristallinité.

6. Matériau en feuille antiadhésif souple selon l'une quelconque des revendications précédentes, ladite couche de composition antiadhésive à base de silicone durcie thermiquement ayant une masse surfacique inférieure à 1,0 g/m$^2$, de préférence de 0,2 g/m$^2$ à 0,8 g/m$^2$, plus préférablement de 0,4 g/m$^2$ à 0,6 g/m$^2$.

7. Matériau en feuille antiadhésif souple selon l'une quelconque des revendications précédentes, ladite couche de composition antiadhésive à base de silicone durcie thermiquement ayant une fraction extractible de moins de 15 %, de préférence de moins de 10 %, plus préférablement de moins de 5 % en poids de ladite composition antiadhésive à base de silicone durcie thermiquement, ladite fraction extractible évaluée selon le procédé « Extractibles dans des revêtements antiadhésifs de silicone » tel que décrit ici.

8. Emballage libérable pour un article hygiénique conditionné individuellement, ledit emballage libérable comprenant un matériau en feuille antiadhésif souple selon l'une quelconque des revendications précédentes.

9. Article hygiénique conditionné individuellement comprenant un article absorbant ayant un côté faisant face au corps, un côté faisant face au vêtement, deux côtés longitudinaux et deux extrémités transversales, ledit article absorbant ayant un élément adhésif sur ledit côté faisant face au vêtement, un emballage libérable se trouvant au-dessus dudit côté faisant face au vêtement dudit article et fixé de manière libérable audit élément adhésif, ledit article absorbant et ledit emballage étant pliés en tant qu'une unité autour d'au moins un axe de pliage pour définir un conditionnement composant ledit article absorbant, dans lequel ledit emballage libérable est selon la revendication 8.

10. Procédé de fabrication d'un matériau en feuille antiadhésif souple selon l'une quelconque des revendications 1 à 7, comprenant les étapes consistant à : fournir un matériau en feuille comprenant au moins 50 % en poids de polyéthylène ayant au moins 10 % de cristallinité, fournir audit matériau en feuille un revêtement d'un polymère de silicone thermodurcissable approprié, durcir ledit polymère de silicone en fournissant de la chaleur.

Fig.1

Fig.2

EP 2 103 664 B2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

• US 4324246 A, Mullane